# EUROPEAN PATENT APPLICATION

(11) **EP 3 943 025 A2**
(43) Date of publication of application: **26.01.2022**
(21) Application number: 21184611.8
(22) Date of filing: 08.07.2021
(51) Int. Cl.: A61B 17/32, A61B 18/14, A61B 17/00, A61B 18/00

(54) **SURGICAL DEVICES, SYSTEMS, AND METHODS PROVIDING VISUAL NOTIFICATIONS**

(30) Priority: 08.07.2020 US 202063049402 P; 09.06.2021 US 202117343371
(71) Applicant: Covidien LP, Mansfield, MA 02048 (US)
(72) Inventor: LYONS, Michael, B, Boulder, CO Colorado 80301 (US); COWLEY, Matthew, S, Boulder, CO Colorado 80301 (US); VAN TOL, David, J, Boulder, CO Colorado 80301 (US)
(74) Representative: Maschio, Antonio

(57) **Abstract**

A surgical apparatus includes an end effector having a blade defining a lumen extending through at least a portion of the blade, a cooling system configured to circulate cooling fluid through the lumen, a temperature sensor configured to sense a temperature of the blade, a visual indicator disposed at the end effector and configured to radiate light, and a controller configured to control the visual indicator based on the sensed temperature of the blade. The cooling system includes an inflow conduit disposed in communication with the lumen and configured to supply the cooling fluid to the lumen and a return conduit disposed in communication with the lumen and configured to receive the cooling fluid from the lumen.

## Description

### CROSS-REFERENCE TO RELATED APPLICATIONS

This application claims the benefit of, and priority to, U.S. Provisional Patent Application No. 63/049,402, filed on July 8, 2020, the entire contents of which are hereby incorporated herein by reference.

### FIELD

The present disclosure relates generally to surgical devices, systems, and methods providing visual notifications and, in particular, to surgical devices, systems, and methods for providing visual cues during a surgical operation based on, for example, the temperature of the surgical instrument (or a portion thereof), a type of tissue being treated, etc.

### BACKGROUND

Surgical devices apply various types of energy (e.g., electrical, ultrasonic, microwave, cryogenic, thermal, laser, etc.) to treat tissue to achieve a desired result. Ultrasonic energy, for example, may be delivered to tissue to treat (e.g., coagulate, cut, seal, ablate, etc.) tissue. While performing such treatments, surgical devices generate heat. However, the generated heat and/or hot surgical device pose a potential risk of damaging nearby tissue.

Further, it is important to perform operations on the intended tissue of interest and/or to avoid certain tissues. However, since surgical operations are often performed minimally-invasively, identification of tissue may be challenging.

### SUMMARY

As used herein, the term "distal" refers to the portion that is being described which is further from an operator (whether a human user or a surgical robot), while the term "proximal" refers to the portion that is being described which is closer to the operator. Further, to the extent consistent, any of the aspects described herein may be used in conjunction with any or all of the other aspects described herein.

Provided in accordance with aspects of the present disclosure is a surgical apparatus having an end effector including a blade defining a lumen extending through at least a portion of the blade, a cooling system configured to circulate cooling fluid through the lumen, a temperature sensor configured to sense a temperature of the blade, a visual indicator disposed at the end effector and configured to radiate light, and a controller configured to control the visual indicator based on the sensed temperature of the blade. The cooling system includes an inflow conduit disposed in communication with the lumen and configured to supply the cooling fluid to the lumen and a return conduit disposed in communication with the lumen and configured to receive the cooling fluid from the lumen.

In an aspect of the present disclosure, the controller controls the visual indicator to radiate a first color of light when the sensed temperature of the blade is greater than a predetermined threshold.

In another aspect of the present disclosure, the controller controls the visual indicator to radiate a second color of light when the temperature of the blade is less than or equal to a predetermined threshold.

In still another aspect of the present disclosure, the visual indicator includes a light source configured to radiate the light around an annular perimeter of the blade.

In yet another aspect of the present disclosure, the visual indicator includes a plurality of light sources. In such aspects, controller may control the plurality of light sources to form a plurality of light patterns based on the temperature of the blade. The plurality of light sources may be disposed along a length direction of the blade.

In another aspect of the present disclosure, the temperature sensor is an optical sensor configured to sense the temperature of the blade by detecting thermal radiation from the blade.

In yet another aspect of the present disclosure, the optical sensor is located at a distal end portion of an elongated probe that receives the blade.

In still another aspect of the present disclosure, the end effector further includes a jaw member operationally coupled with the blade.

In yet another aspect of the present disclosure, the optical sensor is disposed on the jaw member.

In still yet another aspect of the present disclosure, the temperature sensor is mounted within or outside the return conduit and configured to sense temperature of the fluid coming from the blade and returning to the return conduit. The temperature sensor may include a thermocouple, thermistor, resistance thermometer, and/or silicone bandgap sensor.

In still yet another aspect of the present disclosure, the visual indicator is a display screen.

Provided in accordance with other aspects of the present disclosure is a surgical apparatus that includes an end effector including a blade, a temperature sensor configured to sense a temperature of the blade, a visual indicator disposed at the end effector and configured to radiate light, and a controller configured to control the visual indicator based on the sensed temperature of the blade.

Another surgical apparatus provided in accordance with aspects of the present disclosure includes an end effector including a pair of jaw members configured to clamp tissue, a sensor configured to sense at least one property of the clamped tissue or energy supplied thereto via at least one jaw of the pair of jaw members, a visual indicator disposed at the end effector and configured to radiate light, and a controller configured to identify a type of the clamped tissue based on the sensed property and to control the visual indicator to radiate the light, which is indicative of the identified tissue type.

In an aspect of the present disclosure, the controller controls the visual indicator to radiate a first color or first pattern when a type of the clamped tissue matches a type of tissue of interest.

In another aspect of the present disclosure, the controller controls the visual indicator to radiate a second color or second pattern when a type of the clamped tissue does not match a type of tissue of interest.

In yet another aspect of the present disclosure, the visual indicator is a display screen.

In yet another aspect of the present disclosure, the visual indicator displays symbols, characters, or signs on the display screen regarding the type of tissue.

In still yet another aspect of the present disclosure, one jaw of the pair of jaw members is an ultrasonic blade.

### BRIEF DESCRIPTION OF DRAWINGS

Various aspects and features of the present disclosure are described hereinbelow with reference to the drawings wherein like numerals designate identical or corresponding elements in each of the several views:
FIG. 1 is a perspective view of a surgical system including different types of surgical devices in accordance with aspects of the present disclosure;
FIG. 2A is a perspective view of a surgical system with a surgical instrument and a cooling system in accordance with aspects of the present disclosure;
FIG. 2B is an enlarged, perspective view of the area of detail indicated as "2B" in FIG. 2A;
FIG. 2C is an enlarged, perspective view of the area of detail indicates as "2C" in FIG. 2A;
FIG. 3 is an enlarged, perspective view of the distal end of the surgical instrument of FIG. 2A;
FIG. 4 is a schematic illustration of the surgical system of FIG. 2A in accordance with aspects of the present disclosure;
FIG. 5 is an exploded, perspective view of another surgical instrument provided in accordance with aspects of the present disclosure;
FIG. 6 is a side, cross-sectional view of a hemostat-style surgical instrument provided in accordance with the present disclosure;
FIG. 7A is a side, cross-sectional view of an end effector with temperature sensors in accordance with aspects of the present disclosure;
FIGS. 7B and 7C are side, cross-sectional views of an end effector including visual indicators in accordance with aspects of the present disclosure; and
FIGS. 8A-8C are plan views of a portion of an end effector including visual indicators in accordance with aspects of the present disclosure.

### DETAILED DESCRIPTION

Surgical systems may include surgical instruments with different types of end effectors, each of which performs a corresponding surgical operation to a target tissue of interest. Some of the end effectors generate heat in order to treat tissue or as a byproduct thereof, thereby heating a portion of the end effector, e.g., a blade. When heated, the portion of the end effector may cause unintended damage, e.g., to tissue. By providing a notification of a temperature of the portion of the end effector, such damage can be prevented. In particular, visual notifications may be helpful in the typically noisy surgical environment to help ensure the notifications are recognized.

In addition, minimally-invasive and obstructed-view surgeries render the end effector and/or surrounding tissue invisible to the user's naked eyes and even a surgical camera. Further, even when the tissue can be seen, the appearance of other surrounding tissues or organs might be similar to that of the tissue of interest. Thus, identification, confirmation, and/or notification of tissue in contact with, operably positioned relative to, or surrounding the end effector may be difficult but important. In particular, visual notifications may be helpful in the typically noisy surgical environment to help ensure the notifications are recognized.

Although various aspects of the present disclosure are detailed hereinbelow implemented in particular surgical instruments, systems, and/or end effectors thereof, it is understood that the aspects and features of the present disclosure, to the extent consistent, may be utilized with any of the surgical instruments, systems, and/or end effectors detailed herein and/or any other suitable surgical instruments, systems, and/or end effectors.

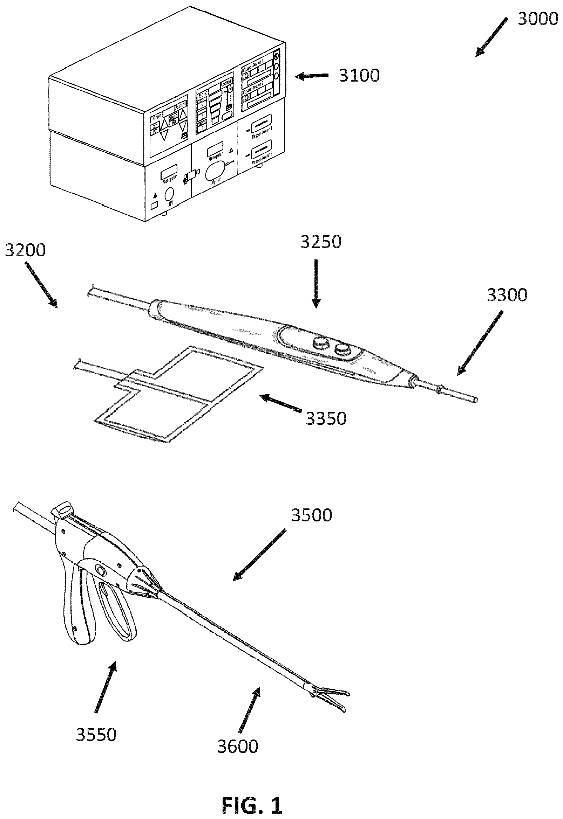

Referring to FIG. 1, a surgical system 3000 may include a surgical generator 3100 and one or more surgical instruments, e.g., a monopolar surgical device 3200, and/or a bipolar surgical device 3500. The monopolar surgical device 3200 may have a handpiece assembly 3250, an elongated probe or end effector assembly 3300 for treating (e.g., cutting, ablating, sealing, coagulating, etc.) tissue, and a return pad 3350.

The monopolar surgical device 3200 may be connected to the surgical generator 3100 via one of several output connectors thereof. The surgical generator 3100 generates surgical energy in the form of radio frequency (RF) energy, although other suitable energies, such as, thermal, microwave, ultrasonic, light, etc., are also contemplated. The surgical energy is supplied to the monopolar surgical device 3200, which applies the surgical energy to treat the tissue via the elongated probe 3300. The surgical energy is then returned to the surgical generator 3100 through the return pad 3350. The return pad 3350 provides a sufficient contact area with the patient's tissue so as to minimize the risk of tissue damage due to the surgical energy applied to the tissue.

The bipolar surgical device 3500 may include a handpiece assembly 3550 and an elongated probe or end effector assembly 3600. The handpiece assembly 3550 may be also connected to the surgical generator 3100 via one of several output connectors. The surgical energy is supplied to one of the two jaw members of the elongated probe 3600 to treat the tissue and is returned to the surgical generator 3100 through the other one of the two jaw members, although other energy modalities are also contemplated such as those noted above.

The surgical generator 3100 may be any suitable type of generator and may include a plurality of connectors to accommodate various types of surgical devices (e.g., monopolar surgical device 3200 and bipolar surgical device 3500). The surgical generator 3100 may also be configured to operate in a variety of modes, such as ablation, cutting, coagulation, and sealing. The surgical generator 3100 may include a switching mechanism (e.g., relays) to switch the supply of RF energy among the connectors to which various surgical devices may be connected. For example, when the monopolar surgical device 3200 is connected to the surgical generator 3100, the switching mechanism switches the supply of RF energy to the monopolar plug. In aspects, the surgical generator 3100 may be configured to provide RF energy to a plurality of surgical devices simultaneously.

The surgical generator 3100 includes a user interface having suitable user controls (e.g., buttons, activators, switches, or touch screens) for providing control parameters to the surgical generator 3100. These controls allow the user to adjust parameters of the surgical energy (e.g., the power level or the shape of the output waveform) so that the surgical energy is suitable for a particular surgical procedure (e.g., coagulating, ablating, sealing, or cutting). The energy delivery devices 3200 and 3500 may also include a plurality of user controls. In addition, the surgical generator 3100 may include one or more display screens for displaying a variety of information related to operation of the surgical generator 3100 (e.g., intensity settings and treatment complete indicators).

The monopolar or bipolar device 3200, 3500 receives energy generated by the surgical generator 3100 and measures current and voltage, which are sensed at the input terminal, where the generated energy is received from the generator 3100, or at the output terminal, where the generated energy is supplied to the tissue of interest from the monopolar or bipolar surgical device 3200, 3500. The measured voltage and current may be different between when the monopolar or bipolar surgical device 3200, 3500 does not touch tissue and when the energy delivery device grasps or touches it. Further, the measured voltage and current may be also different from one type of tissue to another. In this regard, the energy delivery device 3200, 3500 may include a controller (which is not shown), which computes the measurements and determines a type of tissue it touches. Thus, by measuring the voltage and current (and/or other electrical properties such as impedance, power, etc.), a type of tissue, which the energy delivery device grasps or touches, may be identified. Details for identification of a type of tissue in this manner may be found in U.S. Patent No. 8,221,418. Other suitable methods and/or mechanisms for identification of a type of tissue, e.g., vascular, muscle, organ, etc., are also contemplated such as, for example, based on sensor readings indicating one or more properties of tissue, e.g., tissue compressibility, tissue water motility, tissue clarity, tissue color, tissue density, electrical properties of tissue, chemical properties of tissue, etc.

In an aspect, the monopolar or bipolar device 3200, 3500 may include a memory (not shown), which stores a table describing the type of tissue with respect to the measurements of voltage and current. Further, the memory also includes changes in voltage and current with respect to changes in temperature of the tissue.

In another aspect, the memory, which stores the table describing the type of tissue with respect to the measurements of voltage and current, may be included in the generator 3100 of the surgical system 3000.

The surgical system 3000 may be a robotic system and the handpieces 3250, 3550 of the monopolar and bipolar surgical devices 3200, 3500 replaced with suitable hubs (not shown) for connection to the surgical generator 3100 and a robotic device, e.g., a robotic arm.

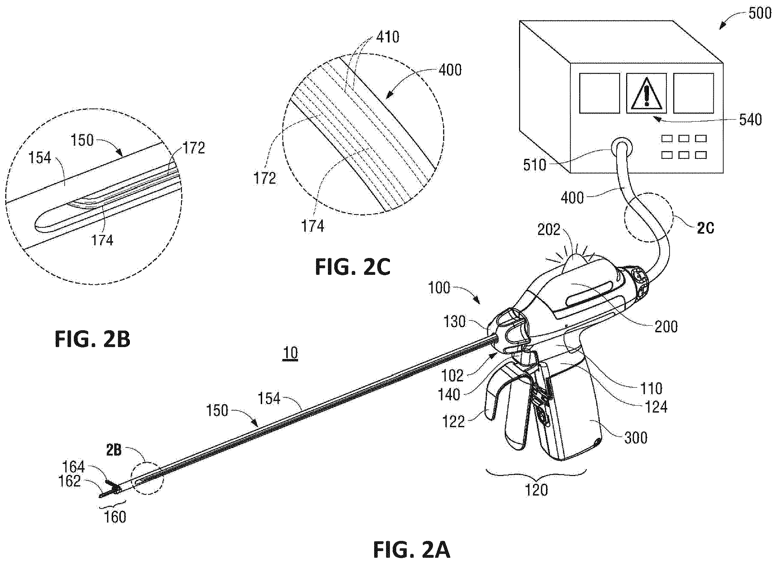

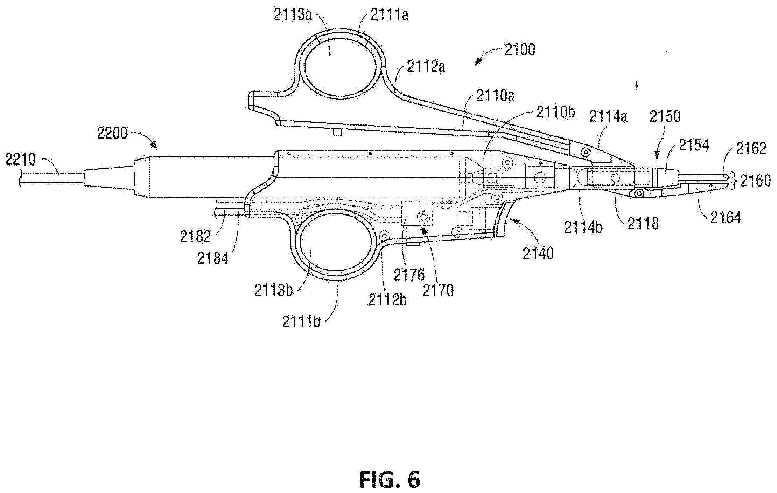

With reference to FIGS. 2A-4, a surgical system 10 includes a cooling module 500 provided in accordance with the aspects of the present disclosure. The surgical system 10 generally includes an energy delivery instrument (e.g., ultrasonic surgical instrument) 100 and the cooling module 500 for cooling a blade 162 of an end effector assembly 160 of the ultrasonic surgical instrument 100. Although detailed hereinbelow with respect to the surgical system 10 and, more particularly, the ultrasonic surgical instrument 100 and the cooling module 500 thereof, the aspects and features of the present disclosure are equally applicable for use with any other suitable surgical systems, surgical instruments, and/or cooling modules. For example, the aspects and features may be provided for use in connection with a surgical system including an ultrasonic surgical instrument 2100 incorporating a cooling module 2500 thereon (see FIG. 6). Obviously, different considerations apply to each particular type of system, instrument, and/or unit; however, the aspects and features of the present disclosure are equally applicable and remain generally consistent with respect to any such system, instrument, and/or unit.

Continuing with reference to FIGS. 2A-4, the ultrasonic surgical instrument 100 generally includes a handset 102, a transducer and generator assembly ("TAG") 200, a battery 300, and a cable 400. The handset 102 includes a housing 110, a handle assembly 120, a rotating assembly 130, an activation button 140, an elongated body portion 150, and end effector assembly 160. The TAG 200 and the battery 300 are releasably engageable with the housing 110 of the handset 102 and, when engaged therewith, are disposed in electrical communication with one another such that power and/or control signals can be relayed between the TAG 200 and the battery 300 for operating the surgical instrument 100.

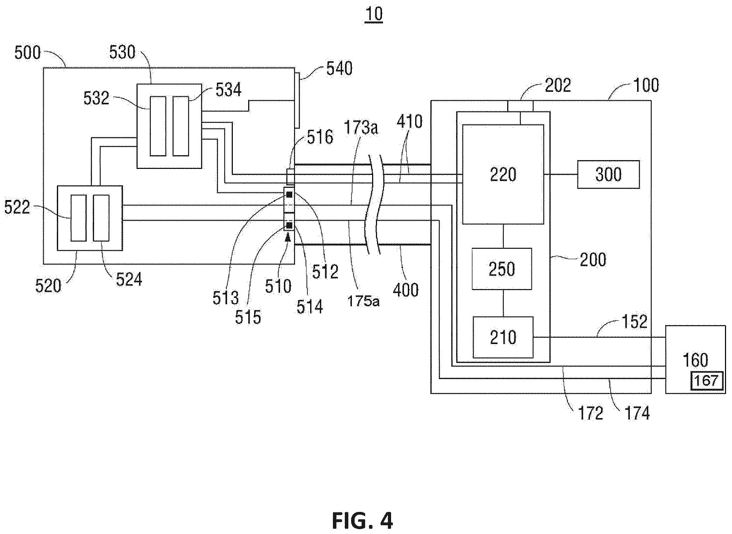

The TAG 200 may include a transducer 210, a generator 220 (FIG. 4), and a controller 250 (FIG. 4). As an alternative to on-board generator 220, ultrasonic surgical instrument 100 may be coupled with a remote generator (e.g., the generator 3100 of FIG. 1). The generator 220 generates and supplies a suitable drive signal to the transducer 210 which, in turn, generates mechanical movements based on the applied drive signal. The controller 250 may control the generator 220 to generate mechanical movements of the transducer 210 with a suitable frequency and/or amplitude. When the frequency of the movements of the transducer 210 is not a resonant frequency of the transducer 210, the controller 250 changes the control parameters of the generator 220 so that the generator 220 generates a drive signal resulting in a suitable frequency of mechanical movement output by the transducer 210. Further, the controller 250 may control the drive signal output by the generator 220 to generate an appropriate amplitude of the mechanical movements output by the transducer 210 to achieve a desired surgical effect.

The TAG 200 may further include a visual indicator 202 disposed thereon, which is capable of producing a plurality of colors of lights. The visual indicator 202 may have one light source, which can produce the plurality of colors of light, or include a plurality of light source, each of which may produce one or more colors of light. The controller 250 may check the temperature of the blade 162 of the end effector assembly 160 and control the visual indicator 202 to produce a color of light based on the temperature of the blade 162. Further, the controller 250 may identify a type of tissue that the blade 162 is in contact with and control the visual indicator 202 to produce a color of light based on the type of tissue. The controller 250 may control the visual indicator 202, in any of the above implementations, to produce a pattern of light together with colors.

For example, where the identified type of the tissue matches a type of target tissue input into the controller 250 or where the identified type of the tissue is a tissue that is acceptable for tissue treatment with instrument 100, the controller 250 may control the visual indicator 202 to display a green color. When the identified type does not match the type of the target tissue input into the controller 250 or where the identified type of the tissue is not acceptable for tissue treatment with instrument 100, the controller 250 may control the visual indicator 202 to display a red color. Different combination of colors and/or other protocols for displaying different colors in response to different tissue types are also contemplated.

Further, a pattern of lighting may be displayed based on the type of tissue. For example, when the identified type of the tissue matches the type of tissue input or is acceptable for treatment, the controller 250 may control the visual indicator 202 to display a solid light. When the identified type does not match the type of the tissue input or is not acceptable for treatment, the controller 250 may control the visual indicator 202 to blink the light. Different patterns of light and/or other protocols for displaying different patterns of light in response to different tissue types are also contemplated. The other visual indicators detailed herein may be configured in a similar manner as detailed here with respect to visual indicator 202, although other configurations are also contemplated.

Continuing with reference to FIGS. 2A-4, the elongated body portion 150 of the handset 102 of the surgical instrument 100 includes a waveguide 152 which extends from the housing 110 to the end effector assembly 160, an outer tube 154, and an inner tube (not shown). The distal end of waveguide 152 extends distally from outer tube 154 and defines blade 162 of end effector assembly 160, while the proximal end of the waveguide 152 is operably coupled to the transducer 220 of the TAG 200. The outer tube 154 is slidably disposed about the waveguide 152 and extends between the housing 110 and the end effector assembly 160. The rotating assembly 130 is rotatably mounted on the housing 110 and operably coupled to the elongated body portion 150 so as to enable rotation of the elongated body portion 150 and the end effector assembly 160 relative to the housing 110.

The end effector assembly 160 is disposed at a distal end of the elongated body portion 150 and includes the blade 162 and a jaw member 164. The jaw member 164 is pivotable relative to the blade 162 between an open position, wherein the jaw member 164 is spaced-apart from the blade 162, and a closed position, wherein the jaw member 164 is approximated relative to the blade 162 in juxtaposed alignment therewith for clamping tissue therebetween. The jaw member 164 is operably coupled to the distal end of the outer tube 154 and the proximal end of the outer tube 154 is operably coupled to a movable handle 122 of the handle assembly 120, such that the jaw member 164 is movable between the open position and the closed position in response to actuation of the movable handle 122 of the handle assembly 120 relative to the fixed handle portion 124 thereof.

The blade 162 is configured to serve as an active or oscillating ultrasonic member that is selectively activatable to ultrasonically treat tissue grasped between the blade 162 and the jaw member 164. The TAG 200 is configured to convert electrical energy provided by the battery 300 into mechanical energy that is transmitted along the waveguide 152 to the blade 162. More specifically, the generator 220 of the TAG 200 is configured to convert the electrical energy provided by the battery 300 into a high voltage alternating current (AC) waveform drive signal that drives the transducer 210 of the TAG 200. The activation button 140 is disposed on the housing 110 of the handset 102 and is electrically coupled between the battery 300 and the TAG 200. The activation button 140 is selectively activatable in a first position and a second position to supply electrical energy from the battery 300 to the TAG 200 for operating the surgical instrument 100 in a low-power mode of operation and a high-power mode of operation, respectively, although alternative activation configurations and/or plural activation buttons 140 are also contemplated.

Continuing with reference to FIGS. 2A-4, cooling inflow and return conduits 172, 174 may extend from the cooling module 500, through the housing 110, and at least partially through the outer tube 154 of the elongated body portion 150 substantially along the length thereof. The proximal ends 173a, 175a of the inflow and return conduits 172, 174, respectively, are operably coupled to the cooling module 500, as detailed below (see also FIG. 4).

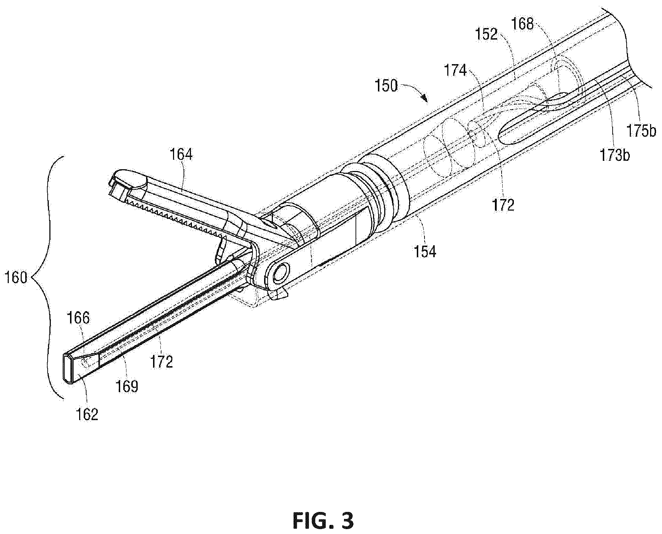

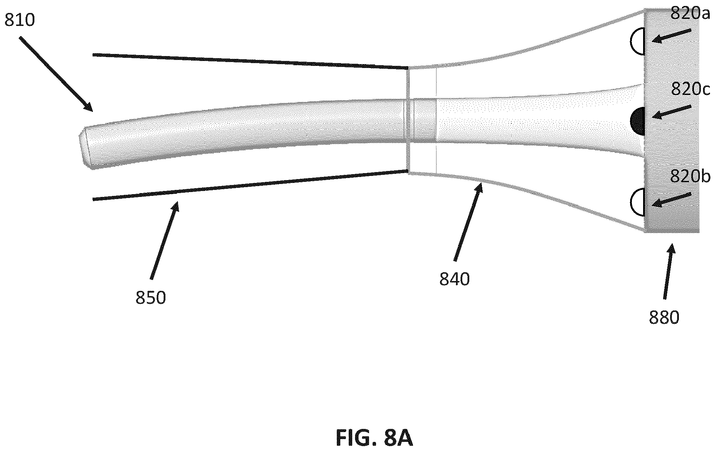

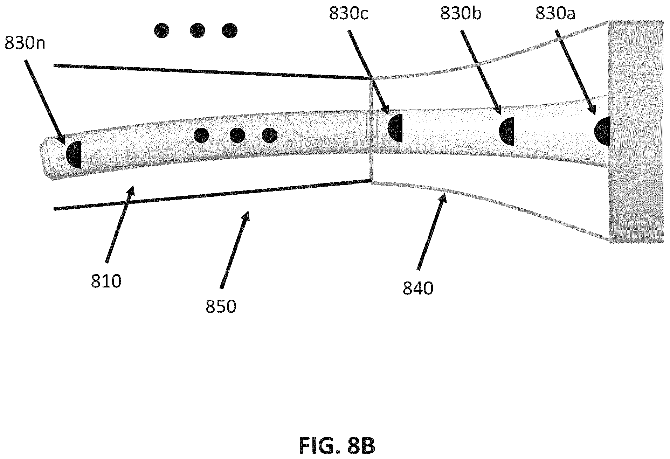

With particular reference to FIG. 3, the distal ends 173b, 175b of the cooling inflow and return conduits 172, 174, respectively, may extend into the waveguide 152. More specifically, a lumen 166 is formed within the waveguide 152 that extends through a portion of the waveguide 152 including substantially along the length of the blade 162 of the waveguide 152. The lumen 166 defines a closed distal end. The cooling inflow and return conduits 172, 174 enter the lumen 166 through an opening 168 defined within the waveguide 152 and disposed in communication with the lumen 166. A seal (not shown) disposed within the opening 168 and around the cooling inflow and return conduits 172, 174 is provided to inhibit the escape of fluid therefrom. The cooling inflow conduit 172 may be disposed within and extends distally through the lumen 166. The return conduit 174 may be disposed within the proximal end of the lumen 166, although the above-detailed configuration of the cooling inflow and return conduits 172, 174 may be reversed. The inflow conduit 172 has a smaller diameter than the lumen 166 leaving an annular gap 169 therebetween to permit the return of fluid to the return conduit 174. As such, during cooling, fluid, e.g., water, saline, etc., is pumped through the cooling inflow conduit 172, exits a distal end of the cooling inflow conduit 172 at the distal end of the lumen 166, and travels proximally back through the lumen 166 within the annular gap 169, ultimately being received by the return conduit 174 for return to the cooling module 500 (FIG. 2). In aspects where the blade 162 is curved, the portion of the cooling inflow conduit 172 that extends distally through the lumen 166 is likewise curved, as shown in FIGS. 8A and 8B, so as not to rub on the interior surface of the blade 162.

In an aspect, the cooling inflow and return conduits 172, 174 may be incorporated into one flow conduit, e.g., one-way conduit through the entire blade. In this case, a push pump may be used to push the cooling fluid into the one-way conduit.

The cooling fluid provided to the blade 162 cools down the blade 162 after use to inhibit damage caused by inadvertent contact with a hot blade, e.g., upon withdrawal, repositioning, or other manipulation thereof. The temperature of the blade 162 may be measured directly at the blade 162, the jaw member 164, and/or a distal end portion of the elongated body portion 150 by a temperature sensor, or indirectly measured at the return conduit 174 (or other suitable location along the return path).

Referring to FIG. 4, the cooling module 500 includes an input port 510, a pump assembly 520, a controller 530, and a user interface 540. The input port 510 enables operable coupling of the cable 400 with the cooling module 500. More specifically, the input port 510 includes an inflow conduit receptacle 512, a return conduit receptacle 514, and one or more electrical receptacles 516. The inflow conduit receptacle 512 operably couples the cooling inflow conduit 172 with the pump assembly 520 upon engagement of the cable 400 with the cooling module 500, the return conduit receptacle 514 operably couples the return conduit 174 with the pump assembly 520 upon engagement of the cable 400 with the cooling module 500, and the electrical receptacles 516 operably couple the TAG 200 with the controller 530, via the wires 410, upon engagement of the cable 400 with the cooling module 500. The cooling inflow and return conduit receptacles 512, 514 each include one or more temperature sensors 513, 515, respectively, associated therewith for sensing the temperature of fluid flowing therethrough, the flow rate of fluid therethrough, and/or the presence of gas bubbles flowing therethrough, as detailed below.

The pump assembly 520 includes a fluid reservoir 522 and a pump 524 and is coupled between the cooling inflow conduit 172 and the return conduit 174. The fluid reservoir 522 stores fluid to be circulated through the conduits 172, 174 and the lumen 166 to cool the blade 162 of the end effector assembly 160 (see FIG. 3) after use. In some configurations, the fluid reservoir 522 may be configured to regulate the temperature of the fluid retained therein. Further, instead of a closed system utilizing the fluid reservoir 522, an open system may be provided wherein fluid to be circulated is received from an external fluid source (not shown), and fluid returning is output to a drain or return reservoir (not shown) or is expelled into the surgical site.

The pump 524 is configured as a pull-pump or push-pump, wherein the pump 524 operates to pull fluid through the conduits 172, 174 and the lumen 166. However, in some aspects, the pump 524 may be configured as a push-pump. The pump 524 may be a peristaltic pump, or any other suitable pump.

Continuing with reference to FIG. 4, the controller 530 of the cooling module 500 includes a processor 532 and a memory 534 storing instructions for execution by the processor 532. The controller 530 is coupled to the pump assembly 520, the temperature sensors 513, 515, the TAG 200 (via the wires 410 extending through the cable 400), and the user interface 540. The controller 530 may be configured to instruct the pump assembly 520 to turn the pump 524 ON or OFF, to thereby initiate or stop blade cooling, based at least upon feedback received from the temperature sensors 513, 515, TAG 200, or other received feedback.

The controller 532 may be configured so as to instruct the pump assembly 520 to turn OFF the pump 524 where the sensor 515 indicates a sufficiently low temperature of fluid returning from the return conduit 174. The temperature of blade 162 (FIG. 3) of the end effector assembly 160 may be extrapolated from the temperature of fluid returning from the return conduit 174, or the temperature difference between the fluid entering the cooling inflow conduit 172 and the fluid returning to the return conduit 174, and, accordingly, the pump 524 may be turned OFF upon the blade 162 reaching a sufficiently cool temperature, e.g., below about 60°C (or other suitable temperature threshold), as indicated by a sufficiently low temperature of the return fluid or sufficiently small temperature differential. As an alternative to the temperature sensors 513, 515 sensing temperature at the input port 510, the temperature sensors 513, 515 may be incorporated into the pump assembly 520 for similar purposes as noted above. Further, in aspects, a thermocouple 167 (FIG. 4) or other suitable temperature sensor may additionally or alternatively be incorporated into the blade 162 to enable the sensing of temperature at the blade 162. The controller 530 may additionally or alternatively instruct the pump assembly 520 to turn OFF the pump 524 or disable the entire system where the temperature sensor 513 and/or 515 indicates an error. In an aspect, the surgeon may be able to override the controller 530, manually turn off the pump assembly 520, or disable the entire system where the temperature sensor 513 and/or 515 indicates an error.

The controller 530 may further be configured to output an appropriate signal to the user interface 540 and/or the visual indicator 202 of the TAG 200 to alert the user that blade cooling is in effect, e.g., that the pump 524 is ON, that an error, e.g., a blockage or leakage, has occurred, and/or that the blade 162 (FIG. 3) has been sufficiently cooled down. The user interface 540 and/or the visual indicator 202 may provide such an alert in the form of audible, visual, and/or tactile output. In a case when the surgical environment is noisy, visual indicators such as those detailed herein may be used.

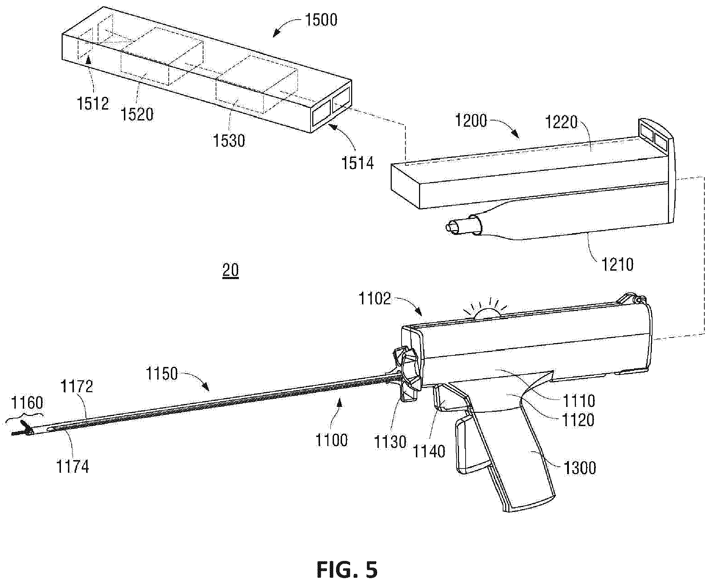

Turning now to FIG. 5, another surgical system 20 is similar to the surgical system 10 (FIG. 2A) and generally includes an ultrasonic surgical instrument 1100 and a cooling module 1500. However, rather than being coupled via a cable 400 as with the ultrasonic surgical instrument 100 and the cooling module 500 (FIG. 1), the surgical instrument 1100 includes the cooling module 1500 disposed therein, e.g., formed as part of the handset 1102 or releasably mounted thereon or therein.

The surgical instrument 1100 generally includes a handset 1102, a TAG 1200 including a transducer 1210 and a generator 1220, and a battery 1300. The handset 1102 includes a housing 1110, a handle assembly 1120, a rotating assembly 1130, an activation button 1140, an elongated body portion 1150, and an end effector assembly 1160, each of which are similar to the corresponding components of the surgical instrument 100 (FIG. 2A), detailed above. The TAG 1200 and the battery 1300 are releasably engageable with the housing 1110 of the disposable 1102 and, when engaged therewith, are disposed in electrical communication with one another such that power and/or control signals can be relayed between the TAG 1200 and the battery 1300 for operating the surgical instrument 1100. The TAG 1200 and the battery 1300 are similar to those detailed above with respect to the surgical instrument 100 (FIG. 2A), except as otherwise noted below.

The cooling module 1500, similar as with the cooling module 500 (FIG. 4), includes input ports 1512, 1514, a pump assembly 1520, and a controller 1530. The cooling module 1500 may be permanently mounted on the TAG 1200, may be releasably engageable with both the TAG 1200 and the disposable 1102, or may be permanently mounted on or within the disposable 1102. The input port 1512 enables operable coupling of the pump assembly 1520 with the conduits 1172, 1174 of the surgical instrument 1100, while the input port 1514 enables communication between the controller 1530 and the generator 1220, both of which are similar as detailed above with respect to the input port 510 (FIG. 4). The pump assembly 1520 and the controller 1530 are also similar as detailed above, and may be configured to operate in a similar manner as mentioned above.

Turning now to FIG. 6, another instrument provided in accordance with the present disclosure, a hemostat style ultrasonic surgical instrument 2100, is detailed. The ultrasonic surgical instrument 2100 is configured to operably coupled to a table-top generator (or other remote generator) (not shown) and a cooling module (similar to cooling module 500). In some configurations, the generator and the cooling module are integrated into a single housing (not shown). The ultrasonic surgical instrument 2100 generally includes two ring handles 2110a, 2110b, an activation button 2140, an elongated body portion 2150, an end effector assembly 2160, a tube assembly 2170, and a transducer assembly 2200.

Each ring handle 2110a, 2110b includes a handle 2111a, 2111b disposed at the proximal end 2112a, 2112b thereof. Each handle 2111a, 2111b defines a finger hole 2113a, 2113b therethrough for receiving a finger of the user. One of the elongated probes, e.g., ring handle 2110a, includes a jaw member 2164 of end effector assembly 2160 extending from the distal end 2114a thereof. The other elongated probe, e.g., the elongated probe 2110b, supports the elongated body portion 2150 and the transducer assembly 2200 thereon. The ring handles 2110a, 2110b are pivotably coupled to one another towards the distal ends 2114a, 2114b, respectively, thereof via a pivot pin 2118. The location of the pivot pin 2118 may be anywhere the ring handles 2110a and 2110b are overlapped or structurally connected.

The elongated body portion 2150 of the elongated probe 2110b includes a waveguide which extends from the transducer assembly 2200 to the end effector assembly 2160, and an outer sleeve 2154 surrounding the waveguide. The distal end of waveguide extends distally from the outer sleeve 2154 and defines a blade 2162 of the end effector assembly 2160, while the proximal end of the waveguide is operably coupled to the transducer assembly 2200. Due to the pivotable coupling of the ring handles 2110a, 2110b towards the distal ends 2114a, 2114b, respectively, thereof, the handles 2111a, 2111b may be pivoted relative to one another to thereby pivot the jaw member 2164 relative to the blade 2162 between an open position, wherein the jaw member 2164 is spaced-apart from the blade 2162, and a closed position, wherein the jaw member 2164 is approximated relative to the blade 2162 in juxtaposed alignment therewith for clamping tissue therebetween.

The transducer assembly 2200 is configured to convert electrical energy provided by the generator (not shown) and supplied via the cable 2210, into mechanical energy that is transmitted along the waveguide to the blade 2162. The transducer assembly 2200 may be permanently affixed to the elongated body portion 2150 or may be removable therefrom. The activation button 2140 is disposed on one of the elongated probes, e.g., elongated probe 2110b, and, similarly as detailed above with respect to the surgical instrument 100 (FIG. 2A), is selectively activatable in a first position and a second position to supply electrical energy to the transducer assembly 2200 for operating the surgical instrument 2100 in a low-power mode of operation and a high-power mode of operation, respectively.

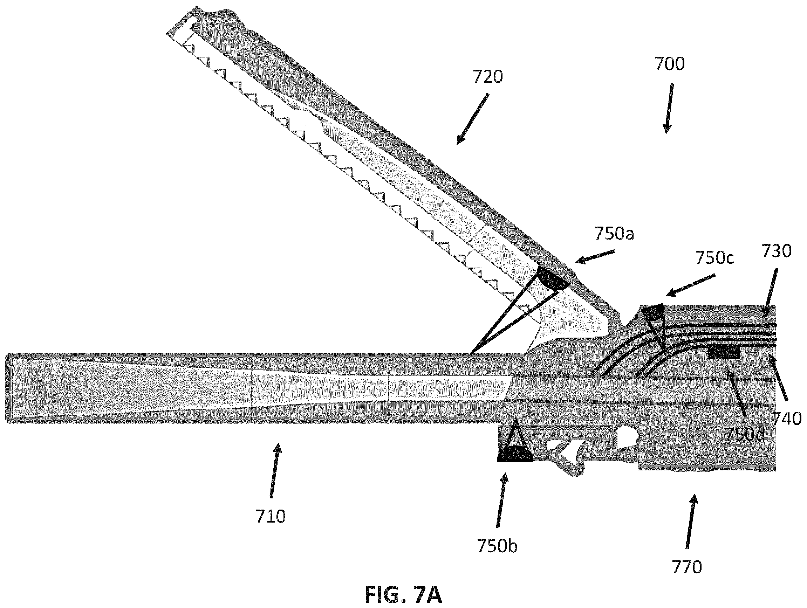

Referring now to FIG. 7A, an end effector 700 of a surgical instrument, according to aspects of the present disclosure includes one or more temperature sensors disposed in one or more different locations thereon. The end effector 700 may be an energy delivery device, such as a bipolar surgical device, a monopolar surgical device, or an ultrasonic surgical device, such as those detailed hereinabove. For example, an ultrasonic surgical end effector of an ultrasonic surgical instrument is shown in FIG. 7A as the end effector 700. The end effector 700 includes two jaw members 710 and 720, one of which (e.g., jaw member 710) is an ultrasonic blade and the other of which (e.g., jaw member 720) is a clamp arm including a more-rigid structural body and a more-compliant jaw liner. The clamp arm 720 is configured to move around a pivot from a first position, which is a spaced apart from the blade 710, to a second position, approximated relative to the blade 710 to grasp tissue between the blade 710 and the jaw liner of the clamp arm 720. When ultrasonic energy is transmitted to the blade 710, the blade 710 is mechanically, e.g., frictionally, heated so as to treat tissue clamped thereto or otherwise in contact therewith.

The blade 710 may be cooled by a cooling fluid. The cooling fluid may be supplied to the blade 710 via an inflow conduit 730 and returned from the blade 710 via a return conduit 740.

The temperature of the blade 710 may be measured by a temperature sensor 750a, which is an optical sensor configured to measure thermal irradiation from the blade 710. Since the optical sensor 750a does not need to touch or physically contact the blade 710, the optical sensor 750 may be installed on the clamp arm 720 opposite the blade 710 or on another suitable component. One of benefits of having the optical sensor 750a on the clamp arm 720 is that it enables direct measurement of the temperature of the blade 710. A plurality of optical sensors 750a installed at spaced-apart positions over at least a portion of the length of the clamp arm 720 may be provided for redundancy measurements, to enable determination of the temperature of different portions of the blade 710, for determining an average temperature along the blade 710, etc.

In an aspect, the temperature of the blade 710 may be also measured by one or more optical temperature sensors 750b located towards the proximal end portion of the blade 710 or at a distal end portion of an elongated probe 770 which receives the blade 710, e.g., a support tube pivotably supporting or an actuator tube movable to actuate the clamp arm 720. However, temperature sensor(s) 750b are not disposed on the blade 710 itself at least because sensitivity of the temperature sensor may be deteriorated due to high temperature of the blade 710. Thus, the temperature sensor(s) 750b is disposed on a portion of the end effector 700 that is in close proximity to the blade 710. In an aspect, the temperature sensor 750b may be a thermocouple located on the blade 710.

In an alternative or additional aspect, the temperature of the blade 710 may be measured by measuring a temperature of the returning cooling fluid by an optical sensor 750c. The optical sensor 750c may be installed near where the return conduit 740 exits the blade 710 and may measure thermal radiation from the return conduit 740, which is heated by the fluid returning from the blade 710. The optical sensor 750c may be a redundancy sensor additional to the optical sensor 750a or 750b or may be provided in place thereof.

In still another aspect, the temperature of the return conduit 740 may be additionally or alternatively mechanically or electrically measured by a sensor 750d, which is installed directly on or in the return conduit 740 near where the return conduit 740 exits the blade 710. The sensor 750d may be a thermocouple, thermistor, resistance thermometer, or silicone bandgap sensor. This list of temperature sensors is provided not as limiting but as examples, and may include other sensors, which are readily contemplated by a person having skill in the art.

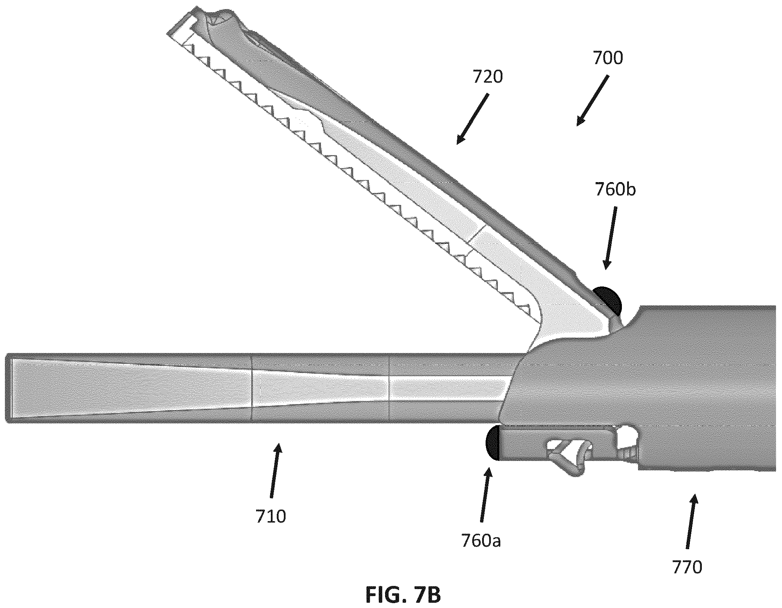

Referring to FIG. 7B, visual indicators 760a, 760b are shown associated with the end effector 700. The first visual indicator 760a may act as a light source to illuminate at least a portion of the blade 710, and/or other portion(s) of the end effector 700, and/or the tissue being treated. The visual indicator 760a is placed on a distal end of the elongated probe 770, e.g., a support tube pivotably supporting or an actuator tube movable to actuate the clamp arm 720, which receives the blade 710. The first visual indicator 760a may be placed in a forward direction so that the radiated light is directed distally along the longitudinal direction of the blade 710. The first visual indicator 760a may have one light source, which can produce a plurality of colors of light, or include a plurality of light source, each of which may produce one or more colors of light; thus the first visual indicator 760a may illuminate a portion of the blade 710, other portion(s) of the end effector 700, and/or the tissue being treated with one or more colors of light. The first visual indicator 760a may additionally or alternatively show different patterns of light. The colors and/or patterns of light provided by the first visual indicator 760a may vary based on types of tissue detected and/or the temperature of the blade 710.

In an aspect, the light source may be positioned in various different directions from the jaw members 740, for example, inwardly onto the blade 710, outwardly away from the blade 710, out to the sides, distal of the jaw member 740, and/or proximal of the jaw member 740. In another aspect, the jaw member 740 may be translucent so that, the visual indicator 760a can be seen any direction even when the blade 710 is positioned toward eyes of the surgeon or a surgical camera and the jaw member 740 is positioned away from the eyes of the surgeon or a surgical camera.

The second visual indicator 760b may be placed on the clamp arm 720 to show colors or patterns of light based on types of tissue detected and/or the temperature of the blade 710, similarly as detailed above with respect to the first visual indicator 760a. The locations of the visual indicators 760a, 760b are examples and additional or alternative visual indicators can be provided on the jaw members 710, 720 or any other suitable positions on or associated with the end effector 700.

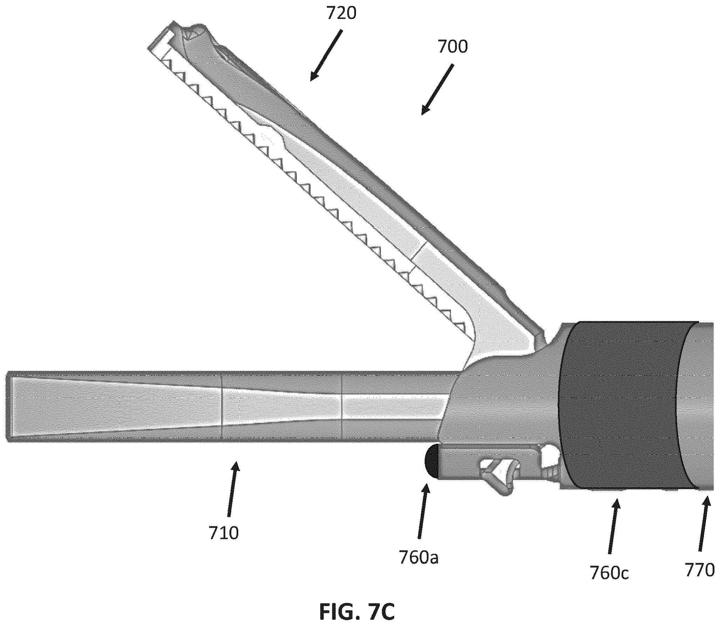

Referring to FIG. 7C, the visual indicator may be a display screen 760c which is capable of showing messages or symbols based on the types of tissue detected and/or the temperature of the blade 710, in accordance with aspects of the present disclosure. The display screen 760c may be affixed on the outer surface of the elongated probe 770 which receives the blade 710. In an aspect, the display screen 760c may function by use of a light pipe or a fiber optic display.

In an aspect, the visual indicator 760c may be flexible to cover the elongated probe 770 about a majority or the entirety of a circumference thereof. The flexible screen 760c may surround the elongated probe 770 so that messages or symbols corresponding to the type of tissue and/or the temperature of the blade 710 may be displayed in a direction facing the user, which may vary based on the direction of gravity (as a result of a varying orientation and/or position of the end effector 770.

Referring to FIG. 8A, visual indicators 820a-820c are shown associated with a jaw member of an end effector of a monopolar, bipolar, or ultrasonic surgical instrument according to aspects of the present disclosure. As illustrated in FIG. 8A and only by way of example, the jaw member is an ultrasonic blade 810 (although not shown, a clamp arm may also be provided). The visual indicators 820a-820c may be controlled based on the type of the tissue detected and/or the temperature of the blade 810 measured by temperature sensor(s), such as the temperature sensors 750a-750d of FIG. 7A. The visual indicators 820a-820c may be fixedly attached to the distal end portion of the elongated probe 880, which receives the blade 810 at a plurality of positions surrounding an annular perimeter of the blade 810. Thus, the lighting direction of the visual indicators 820a-820c may be substantially in line with the longitudinal direction of the blade 810 around a portion of the entirety of the annular perimeter thereof.

Any suitable number of visual indicators 820a-820c may be provided. At least one of the visual indicators 820a-820c may be a visual cue, which when lit, provides a visual indication corresponding to the temperature of the blade 810 or the type of tissue detected.

Some or all of the visual indicators 820a-820c may be used as illumination light sources. For example, the visual indicators 820a and 820b may radiate light to illuminate the blade 810 and at least a portion of the surroundings of the blade 810. Such lightings provide sufficient illumination of the surroundings so that a sufficiently bright view around the blade 810 can be obtained for visualization, even without use of an external light source. The light may be white light, predetermined frequency light (e.g., laser), fluorescent light, or any other light which can provide illumination to the blade 810 and/or surroundings. Furthermore, the lights may cause target tissue, which has been dyed with special dye, to glow so that medical professionals may easily identify the target tissue. Some or all of the lights may be focused by use of a light pipe in the form of the annular perimeter of the blade 810 or other means so as to illuminate a target. In an aspect, some or all of the light may be dispersed in the annular perimeter of the blade 810 to provide better optic feedback to the user.

Some or all of the visual indicators 820a-820c may additionally or alternatively be used as a visual cue indicating whether the blade 810 is safe to contact and/or the tissue detected is the right type of tissue. For example, the visual indicator 820c may be capable of radiating a plurality of colors of different light based on the temperature of the blade 810 and/or a tissue type detected. A list of colors may be based on a level of attentiveness. For example, when the blade 810 is sufficiently hot that it may cause damage if contacted, the visual indicator 820c may radiate red or orange light indicating potential danger. When the blade 810 is sufficiently cool that it is safe to contact, the visual indicator 820c may radiate green or blue light, indicating that the blade 810 is safe to touch. For example, a temperature of less than 45°C would be considered "cold" and safe to use in any condition and would be shown with a blue light. Whereas, a temperature between 45°C and 60°C would be considered "warm" and safe to touch healthy tissue for a brief moment of time and would be shown with a yellow light. While a temperature of more than 60°C would be considered "hot" and unsafe to use in any condition and would be shown with a red light. The temperature ranges for indicating hot, warm and cold may be different for different tissues or organs. This list of colors is neither extensive nor exhaustive. The choice of colors may be different from place to place, or from surgery to surgery.

In addition or as an alternative to different colors, different light patterns may be provided, different portions of the blade 810 or end effector may be illuminated (or not illuminated), etc. For example, a blinking pattern may be used by the visual indicator 820c wherein, when the blade 810 is too hot for safe contact, the visual indicator 820c blinks rapidly. When the blade 810 is suitable for safe contact, the visual indicator 820c may be turned off, radiate solid light without blinking, or may blink slowly. Other kinds of patterns may be also employed to differentiate temperatures of the blade 810. Further the change in color or pattern associated with any of the visual indicators detailed herein may be continuous, e.g., changing color along the color spectrum from one color to another (for example, from red, through orange, yellow, and green, to blue) or stepped, e.g., changing from one color to another at a threshold.

In aspects, all of the visual indicators 820a-820c may show patterns of light collectively. For example, a predetermined on-off pattern may be used to provide indications. For example, when all visual indicators 820a-820c are on, the blade 810 is too hot for contact; when two of the visual indicators 820a-820c are on, the blade 810 is medium-hot; when only one of the visual indicators 820a-820c is on, then the blade 810 is warm; and when none of the visual indicators 820a-820c is on, then the blade 810 is substantially cooled to its resting temperature (or within a minimal temperature range therefrom, e.g., 10%). Other combination of on-off patterns may be used in a similar way.

Further, a light pipe 840 may surround the proximal portion or all of the blade 810. The light pipe 840 may be made of a material and have a shape so that the light emitted to the light pipe 840 does not escape from the side of the light pipe 840 but escapes at the distal end of the light pipe 840. In other words, when the light emitted from the visual indicators 820a-820c goes through the light pipe 840, the light may be totally reflected from the side of the light pipe 840, escape from the distal end of the light pipe 840, and form a light beam 850 shining and focusing around the blade 810.

In an aspect, the light pipe 840 may be made of a material which evenly or uniformly distribute the light, which is entered into the light pipe 840, so that the surroundings of the light pipe 840 may be shined. For example, the light, which entered in the light pipe 840, may escape every surface of the light pipe 840.

Referring to FIG. 8B, another aspect of visual indicators 820a-820n is shown with the blade 810. The visual indicators 830a-830n are attached on the outer surface of the blade 810 or may be virtual indicators projected thereon. The number of the visual indicators 830a-830n may be greater than or equal to four. A pattern of lights may be formed by the visual indicators 830a-830n. For example, a forward arrowing or distal chasing pattern may be made when the blade 810 is sufficiently cool. The forward arrowing pattern is made when the first visual indicator 830a is sequentially turned on, the second visual indicator 830b is then sequentially turned on, and the last visual indicator 830n is then sequentially turned on, thereby showing the forward arrow or chasing pattern. The visual indicators 830a-830n may be sequentially turned off in a similar manner to achieve the chasing pattern.

In an aspect, the visual indicators 830a-830n may use colors of light, or colors of light and patterns together to provide notifications. For example, the visual indicators 830a-830n may blink red lights to indicate that the blade 810 is too hot for safe contact. The visual indicators 830a-830n may radiate green light with the forward arrowing pattern to indicate that the blade 810 is appropriate for contact. Other patterns of light may also be contemplated.

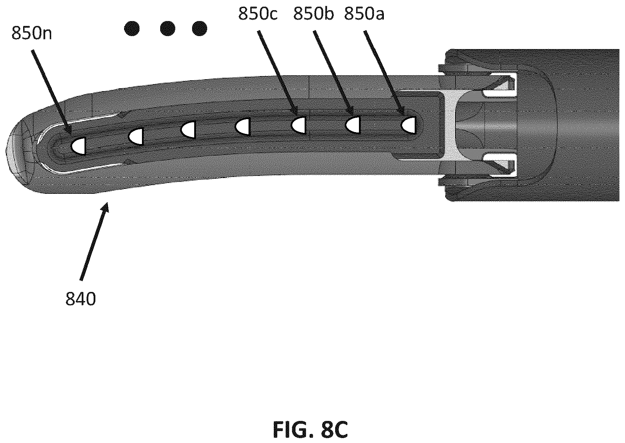

Referring to FIG. 8C, another aspect of visual indicators 850a-850n is shown disposed along a jaw member 840. The jaw member 840 may cooperate with a blade (or other jaw member) as part of an end effector and may be pivotable relative to the blade between an open position, wherein the jaw member 840 is spaced-apart from the blade, and a closed position, wherein the jaw member 840 is approximated relative to the blade in juxtaposed alignment therewith for clamping tissue therebetween. The visual indicators 850a-850n are attached on the back of the jaw member 840, e.g., opposite the blade-contacting surface thereof, or may be virtual indicators projected thereon. The number of the visual indicators 850a-850n may be greater than or equal to four. The pattern of lights including the forward arrowing pattern may be made by the visual indicators 850a-850n in a similar way as detailed above with respect to FIG. 8B.

The visual indicators 850a-850n may use any combinations of colors of light and/or patterns to provide notifications based on the temperature of the blade and/or the type of tissue in a similar way as in FIG. 8B.

The visual indicators of FIGS. 7B-8C may be light sources, light-emitting diodes (LEDs), translucent light pipes, fiber optics, liquid crystal displays (LCD), etc. This list is not meant to be exhaustive but can include other types of light sources, which can show one color or several colors of light. When these light sources can show only one color, each visual indicator may include several light sources, each of which shows a different color from each other. In a case when each light source can show several colors, each visual indicator includes one light source and is controlled by the controller to change colors based on the type of tissue and/or the temperature of the blade.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The invention may be described by reference to the following numbered paragraphs:-
1. A surgical apparatus comprising:
   an end effector including a blade defining a lumen extending through at least a portion of the blade;
   a cooling system configured to circulate cooling fluid through the lumen, the cooling system including:
      an inflow conduit disposed in communication with the lumen and configured to supply the cooling fluid to the lumen; and
      a return conduit disposed in communication with the lumen and configured to receive the cooling fluid from the lumen;
   a temperature sensor configured to sense a temperature of the blade;
   a visual indicator configured to radiate light, the visual indicator disposed at the end effector; and
   a controller configured to control the visual indicator based on the sensed temperature of the blade.
2. The surgical apparatus according to paragraph 1, wherein the controller controls the visual indicator to radiate a first color of light when the sensed temperature of the blade is greater than a predetermined threshold.
3. The surgical apparatus according to paragraph 1, wherein the controller controls the visual indicator to radiate a second color of light when the temperature of the blade is less than or equal to a predetermined threshold.
4. The surgical apparatus according to paragraph 1, wherein the visual indicator includes a light source configured to radiate the light around an annular perimeter of the blade.
5. The surgical apparatus according to paragraph 1, wherein the visual indicator includes a plurality of light sources, and
   wherein the controller controls the plurality of light sources to form a plurality of light patterns based on the temperature of the blade.
6. The surgical apparatus according to paragraph 5, wherein the plurality of light sources is disposed along a length direction of the blade.
7. The surgical apparatus according to paragraph 1, wherein the temperature sensor is an optical sensor configured to sense the temperature of the blade by detecting thermal radiation from the blade.
8. The surgical apparatus according to paragraph 7, wherein the optical sensor is located at a distal end portion of an elongated probe that receives the blade.
9. The surgical apparatus according to paragraph 7, wherein the end effector further includes a jaw member operationally coupled with the blade.
10. The surgical apparatus according to paragraph 7, wherein the optical sensor is disposed on the jaw member.
11. The surgical apparatus according to paragraph 1, wherein the temperature sensor is mounted within or outside the return conduit and configured to sense temperature of the fluid coming from the blade and returning to the return conduit.
12. The surgical apparatus according to paragraph 11, wherein the temperature sensor is at least one of a thermocouple, thermistor, resistance thermometer, or silicone bandgap sensor.
13. The surgical apparatus according to paragraph 1, wherein the visual indicator is a display screen.
14. A surgical apparatus comprising:
   an end effector including a blade;
   a temperature sensor configured to sense a temperature of the blade;
   a visual indicator configured to radiate light, the visual indicator disposed at the end effector; and
   a controller configured to control the visual indicator based on the sensed temperature of the blade.
15. A surgical apparatus comprising:
   an end effector including a pair of jaw members configured to clamp tissue;
   a sensor configured to sense at least one property of the clamped tissue or energy supplied thereto via at least one jaw of the pair of jaw members;
   a visual indicator configured to radiate light, the visual indicator disposed at the end effector; and
   a controller configured to identify a type of the clamped tissue based on the sensed property and to control the visual indicator to radiate the light, which is indicative of the identified tissue type.
16. The surgical apparatus according to paragraph 15, wherein the controller controls the visual indicator to radiate a first color or first pattern when a type of the clamped tissue matches a type of tissue of interest.
17. The surgical apparatus according to paragraph 15, wherein the controller controls the visual indicator to radiate a second color or second pattern when a type of the clamped tissue does not match a type of tissue of interest.
18. The surgical apparatus according to paragraph 15, wherein the visual indicator is a display screen.
19. The surgical apparatus according to paragraph 18, wherein the visual indicator displays symbols, characters, or signs on the display screen regarding the type of tissue.
20. The surgical apparatus according to paragraph 15, wherein one jaw of the pair of jaw members is an ultrasonic blade.

## Claims

1. A surgical apparatus comprising:
an end effector including a blade defining a lumen extending through at least a portion of the blade;
a cooling system configured to circulate cooling fluid through the lumen, the cooling system including:
an inflow conduit disposed in communication with the lumen and configured to supply the cooling fluid to the lumen; and
a return conduit disposed in communication with the lumen and configured to receive the cooling fluid from the lumen;
a temperature sensor configured to sense a temperature of the blade;
a visual indicator configured to radiate light, the visual indicator disposed at the end effector; and
a controller configured to control the visual indicator based on the sensed temperature of the blade.

2. The surgical apparatus according to claim 1, wherein the controller controls the visual indicator to radiate a first color of light when the sensed temperature of the blade is greater than a predetermined threshold.

3. The surgical apparatus according to claim 1 or 2, wherein the controller controls the visual indicator to radiate a second color of light when the temperature of the blade is less than or equal to a predetermined threshold.

4. The surgical apparatus according to claim 1,2 or 3 wherein the visual indicator includes a light source configured to radiate the light around an annular perimeter of the blade.

5. The surgical apparatus according to any preceding claim, wherein the visual indicator includes a plurality of light sources, and
wherein the controller controls the plurality of light sources to form a plurality of light patterns based on the temperature of the blade preferably wherein the plurality of light sources is disposed along a length direction of the blade.

6. The surgical apparatus according to any preceding claim, wherein the temperature sensor is an optical sensor configured to sense the temperature of the blade by detecting thermal radiation from the blade preferably wherein the optical sensor is located at a distal end portion of an elongated probe that receives the blade.

7. The surgical apparatus according to claim 6, wherein the end effector further includes a jaw member operationally coupled with the blade.

8. The surgical apparatus according to claim 6, wherein the optical sensor is disposed on the jaw member.

9. The surgical apparatus according to any preceding claim, wherein the temperature sensor is mounted within or outside the return conduit and configured to sense temperature of the fluid coming from the blade and returning to the return conduit preferably wherein the temperature sensor is at least one of a thermocouple, thermistor, resistance thermometer, or silicone bandgap sensor.

10. The surgical apparatus according to any preceding claim, wherein the visual indicator is a display screen.

11. A surgical apparatus comprising:
an end effector including a blade;
a temperature sensor configured to sense a temperature of the blade;
a visual indicator configured to radiate light, the visual indicator disposed at the end effector; and
a controller configured to control the visual indicator based on the sensed temperature of the blade.

12. A surgical apparatus comprising:
an end effector including a pair of jaw members configured to clamp tissue;
a sensor configured to sense at least one property of the clamped tissue or energy supplied thereto via at least one jaw of the pair of jaw members;
a visual indicator configured to radiate light, the visual indicator disposed at the end effector; and
a controller configured to identify a type of the clamped tissue based on the sensed property and to control the visual indicator to radiate the light, which is indicative of the identified tissue type.

13. The surgical apparatus according to claim 12, wherein the controller controls the visual indicator to radiate a first color or first pattern when a type of the clamped tissue matches a type of tissue of interest.

14. The surgical apparatus according to claim 12 or 13, wherein the controller controls the visual indicator to radiate a second color or second pattern when a type of the clamped tissue does not match a type of tissue of interest preferably wherein the visual indicator is a display screen preferably wherein the visual indicator displays symbols, characters, or signs on the display screen regarding the type of tissue.

15. The surgical apparatus according to claim 14, wherein one jaw of the pair of jaw members is an ultrasonic blade.
